# EUROPÄISCHE PATENTSCHRIFT

(11) **EP 3 513 778 B1**
(45) Veröffentlichungstag und Bekanntmachung des Hinweises auf die Patenterteilung: **11.03.2020**
(21) Anmeldenummer: 18152977.7
(22) Anmeldetag: 23.01.2018
(51) Int. Cl.: A61K 6/00

(54) **PHOTOCHROMER DENTALWERKSTOFF MIT VERKAPSELTEN PHOTOCHROMEN FARBMITTEL**
PHOTOCHROMIC DENTAL MATERIAL WITH ENCAPSULATED PHOTOCHROMIC COLOURANT
MATÉRIAU DENTAIRE PHOTOCHROMIQUE POURVU D'UNE MATIÈRE COLORANTE PHOTOCHROMIQUE ENCAPSULÉE

(43) Veröffentlichungstag der Anmeldung: 24.07.2019
(73) Patentinhaber: Ivoclar Vivadent AG, 9494 Schaan (LI)
(72) Erfinder: BOCK, Thorsten, 6800 Feldkirch (AT); HAUNER, Martina, 6800 Feldkirch (AT)
(74) Vertreter: Uexküll & Stolberg

(56) Entgegenhaltungen:
- EP-A2- 0 744 172
- US-A1- 2006 194 172
- US-B1- 6 531 118
- ZHOU YUEHUA ET AL: "Preparation and application of melamine-formaldehyde photochromic microcapsules", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, Bd. 188, 2013, Seiten 502-512, XP028735335, ISSN: 0925-4005, DOI: 10.1016/J.SNB.2013.07.049
- TIVADAR FECZK ET AL: "Preparation and characterization of photochromic poly(methyl methacrylate) and ethyl cellulose nanocapsules containing a spirooxazine dye", JOURNAL OF PHOTOCHEMISTRY AND PHOTOBIOLOGY, A: CHEMISTRY, ELSEVIER SEQUOIA, LAUSANNE, CH, Bd. 222, Nr. 1, 15. Juni 2011 (2011-06-15) , Seiten 293-298, XP028261402, ISSN: 1010-6030, DOI: 10.1016/J.JPHOTOCHEM.2011.06.011 [gefunden am 2011-06-22]

## Beschreibung

Die vorliegende Erfindung betrifft thermisch- und/oder lichthärtende Dentalwerkstoffe mit lichtinduzierter, reversibler Farbgebung, wie polymerisierbare Harze und Komposite, die sich besonders als dentale Fissurenversiegler, Füllungskomposite, Verblendmaterialien und dentale Beschichtungsmaterialien sowie zur Herstellung von Inlays und Onlays eignen.

Bei Dentalwerkstoffen auf Kunststoffbasis werden dem Polymer Farbmittel, man unterscheidet zwischen Farbstoffen und Pigmenten, zur Einstellung der gewünschten Farbe und Transparenz als Additive zugesetzt. Farbstoffe sind organischer Natur und meist in organischen Lösungsmitteln löslich, während Pigmente feste Teilchen mit Korngrößen zwischen ca. 0,01 bis ca. 1 µm sind und in organische und anorganische Pigmente unterteilt werden (vgl. Taschenbuch der Kunststoff-Additive, Hrsg. R. Gächter, H. Müller, 3. Aufl., Carl Hanser Verlag, München und Wien 1989, 663-736). Bei zahnärztlichen Dentalmaterialien und insbesondere bei hochästhetischen Füllungskompositen werden zur Einstellung der Farbe Mischungen verschiedener anorganischer Pigmente eingesetzt, die sich durch geringe Löslichkeit in organischen Lösungsmitteln und Fetten sowie durch eine sehr gute Farbstabilität auszeichnen. Neben der permanenten Färbung von Dentalmaterialien ist es in manchen Situationen vorteilhaft, das Dentalmaterial temporär durch reversible Farbgebung besser sichtbar machen zu können. Beispiele hierfür sind eine reversible Farbgebung zur Erkennung dünner Schichten, wie im Fall von Fissurenversiegelungen, von mit Adhäsiven behandelten Zahnoberflächen oder von Zementüberschüssen.

Die EP 0 744 172 A2 offenbart photochrome Dentalwerkstoffe, die ein photochromes Material wie beispielsweise einen photochromen Farbstoff, ein photochromes Glas, eine photochrome Keramik oder eine photochrome Glaskeramik enthalten. Der photochrome Dentalwerkstoff lässt sich durch kurze Bestrahlung mit Licht in einen gefärbten Zustand überführen, der eine bessere Unterscheidung von der natürlichen Zahnhartsubstanz möglich macht. Nachteilig ist, dass sich die anschließende Entfärbung zum Teil über mehrere Stunden hinzieht, was die lichtinduzierte Materialaushärtung beeinträchtigen kann. Außerdem kann der Zahnarzt die endgültige Farbgebung der Restauration am Ende der Sitzung nicht abschließend kontrollieren, so dass ein weiterer Besuch des Patienten erforderlich ist.

Bei der Verwendung photochromer Glasfüllstoffe kommt erschwerend hinzu, dass der zur Erzielung eines sichtbaren Farbumschlags erforderliche Füllstoffgehalt von etwa 15% die Viskosität des Werkstoffs deutlich erhöht, so dass derartige Füllstoffe für Anwendungen, die eine niedrige Viskosität erfordern, ungeeignet sind. Durch den relativ hohen Füllstoffgehalt wird zudem die Verwendung weiter Füllstoffe eingeschränkt, z.B. die Verwendung von fluoridfreisetzenden Füllstoffen. Nachteilig ist auch, dass sich photochrome Gläser, deren Farbumschlag in der Regel auf der Verwendung von Silberhalogeniden beruht, bei Lichteinwirkung dunkel färben. Diese Dunkelfärbung lässt sich z.B. bei der Retentionskontrolle von Fissurenversieglern nur schwer von einem Randspalt oder einer durch Karies bedingten Verfärbung unterscheiden, was Fehlinterpretationen begünstigt.

Photochrome Farbstoffe haben andererseits den Nachteil, dass ihre Funktion durch die umgebende Matrix beinträchtig wird. Dabei spielen die Steifigkeit der Matrix, das freie Volumen, Polarität und Hydrophobie, intermolekulare Wechselwirkungen zwischen Matrix und Farbstoff und die Mischbarkeit eine Rolle. Wechselwirkungen zwischen Matrix und Farbstoff oder sterische Hinderungen können die für den Farbübergang verantwortlichen chemischen Vorgänge des photochromen Systems behindern. Dies kann den gewünschten Farbwechsel stark erschweren oder sogar ganz verhindern. Diese Nachteile ließen sich durch den Einsatz flexibler Matrix-Materialien mit einem niedrigen Glasübergangspunkt T_{g} zwar zurückdrängen, diese Möglichkeit scheidet im Falle von Dentalwerkstoffen allerdings aus, weil hierdurch die Härte und Festigkeit der Materialien beeinträchtigt würde. Aus dem gleichen Grund ist der Einsatz von porösen Matrixmaterialien, die dem photochromen Farbstoff eine gewisse molekulare Mobilität bieten, ebenfalls nicht möglich.

Die EP 3 184 096 A1 offenbart radikalisch polymerisierbare Dentalwerkstoffe mit lichtinduzierter reversibler Farbgebung. Die Werkstoffe lassen sich durch kurzzeitiges Bestrahlen mit Licht einer ersten Wellenlänge färben und durch Bestrahlen mit Licht einer zweiten Wellenlänge wieder entfärben. Nachteilig ist, dass Lichtquellen erforderlich sind, die Licht unterschiedlicher Wellenlängen ausstrahlen können, so dass die in Zahnarztpraxen vorhandenen Polymerisationslampen nicht eingesetzt werden können.

Die EP 1 230 906 A1 offenbart Dentalwerkstoffe, die einen thermochromen Farbstoff enthalten und die bei Temperaturänderungen eine reversible Farbänderung zeigen. Dies kann zu unbeabsichtigter Farbgebung in heißer oder kalter Umgebung oder bei Zufuhr von heißen oder kalten Speisen oder Getränken führen.

Aus DE 39 39 998 A1 sind Materialien mit fluoreszierenden Farbstoffen bekannt, die bei der Bestrahlung mit UV-Licht sichtbares Licht emittieren. Da die Fluoreszenz nur während der UV-Einstrahlung auftritt, muss das Arbeitsfeld mit einer zusätzlichen Lichtquelle kontinuierlich bestrahlt werden, was dessen Zugänglichkeit erschwert.

Die US 2006/0194172 A1 offenbart radikalisch polymerisierbare Dentalmaterialien, die neben einem phosphoreszierenden Material zusätzlich ein photochromes Material enthalten können, das in Glas oder Mikroperlen eingekapselt sein kann.

Der Erfindung liegt die Aufgabe zugrunde, photochrome Werkstoffe für dentale Anwendungen bereitzustellen, die nicht die mit dem Stand der Technik verbundenen Nachteile aufweisen. Die Werkstoffe sollen insbesondere einen deutlich sichtbaren Farbeffekt zeigen, der eine klare Unterscheidung von der natürlichen Zahnsubstanz ermöglicht, sie sollen sich schnell entfärben, so dass eine abschließende Beurteilung der Färbung im Verlauf eines Zahnarzttermins möglich ist, und sie sollen dauerhaft photochrome Eigenschaften aufweisen. Eine weitere Aufgabe ist die Bereitstellung photochromer Dentalwerkstoffe mit geringer Viskosität, die sich besonders als Fissurenversiegler eignen. Die Werkstoffe sollen sich mit den üblichen Geräten einer Zahnarztpraxis verwenden lassen.

Die Aufgabe wird erfindungsgemäß durch radikalisch polymerisierbare Dentalwerkstoffe gelöst, die mindestens ein verkapseltes photochromes Farbmittel enthalten.

Unter verkapselten Farbmitteln werden Farbmittel verstanden, die in eine Polymerkapsel eingeschlossen sind. Die Polymerkapseln sind im Unterschied zu Polymerpartikeln nicht massiv, sondern weisen eine äußere Kapselwand und einen inneren Kern auf. Der Kern der Kapseln ist flüssig und enthält das Farbmittel in gelöster oder dispergierter Form. Das Farbmittel liegt vorzugsweise in chemisch ungebundener Form vor.

Die Kapselwand wird durch ein organisches Polymer gebildet. Bevorzugt sind Polymere auf der Basis von Melamin-Formaldehyd-Harzen, Polyurethanen, Polysacchariden, Harnstoff-Formaldehyd-Harzen, Polymethacrylaten und Polymilchsäure. Besonders bevorzugt sind Polymere auf der Basis von Melamin-Formaldehyd-Harzen, wobei Melamin-Formaldehyd-Harz auf der Basis von N-(4,6-Diamino-1,3,5-triazin-2-yl)formamid (CAS-Nr. 13236-84-5) ganz besonders bevorzugt ist. Die Bildung der Kapselwand erfolgt im Fall von Melamin-Formaldehyd-Harzen durch Polykondensation.

Die Kapseln enthalten mindestens ein photochromes Farbmittel, vorzugsweise mindestens einen photochromen organischen Farbstoff oder ein photochromes organisches Pigment. Erfindungsgemäß sind photochrome Farbmittel bevorzugt, die bei der Bestrahlung mit Licht einen Farbumschlag zeigen und die sich anschließend thermisch wieder entfärben.

Geeignete photochrome Farbstoffe werden beispielsweise in Photochromism - Molecules and Systems (Dürr, H.; Bouas-Laurent, H., Herausgeber, Elsevier, 1990) beschrieben. Bevorzugte photochrome Farbstoffsysteme basieren auf der cis/trans-Isomerie von Azobenzolverbindungen oder Stilbenen, der Interkonversion oder elektrocyclischen Ringschluß-/Ringöffnungsreaktion von Spiropyran-Systemen oder Spirooxazinen zu Merocyaninen, oder der 1,5-Elektrocyclisierung von Pentadienyl-Anionen.

Eine bevorzugte Gruppe photochromer Farbstoffe sind Spiro[1,8a-indolizin]-Derivate, insbesondere Spiro[1,8a-dihydro-indolizin]- und Spiro[1,8a-tetrahydroindolizin]-Derivate. Geeignete Derivate und Verfahren zu deren Herstellung werden beispielsweise in der DE 29 O6 193 C2 und der DE 32 20 257 C2 offenbart.

Weiter bevorzugt sind Systeme, die auf einer 1,5-Elektrocyclisierung basieren, wie sie von H. Dürr in Angew. Chem. 101 (1989), Seiten 427 bis 445 in Kapitel 3 beschrieben werden.

Ebenso bevorzugt sind Spiro[fluoren-9,1'[1,8a]dihydro-indolizin]-Derivate, insbesondere Derivate gemäß der Formel I wie in EP 0 744 172 A2 definiert.

Besonders bevorzugte photochrome Farbstoffe sind Spirooxazine, insbesondere solche mit dem Grundkörper 1,3,3-Trimethyl-spiro[indolino-2,3'-[3H]napht[2,1-b][1,4]oxazin (NISO). Ein ganz besonders bevorzugter Farbstoff dieses Typs ist 6'-Piperidin-1,3,3,-trimethyl-spiro[indolino-2,3'-[3H]naphth[2,1-b][1,4]oxazin] (CAS-Nr. 114747-45-4; Reversacol Plum Red).

Die erfindungsgemäß verwendeten photochromen Farbstoffe zeichnen sich durch eine reversible lichtinduzierte Farbgebung aus, d.h., sie lassen sich durch kurzzeitiges Bestrahlen mit Licht der Wellenlänge λ₁ färben, und sie entfärben sich nach Beendung der Bestrahlung bei physiologischen Bedingungen, wie sie z.B. in der Mundhöhle vorliegen, durch eine thermisch bedingte Rückreaktion.

Die dabei ablaufenden Reaktionen sind beispielhaft für Farbstoffe mit dem NISO-Grundkörper gezeigt:

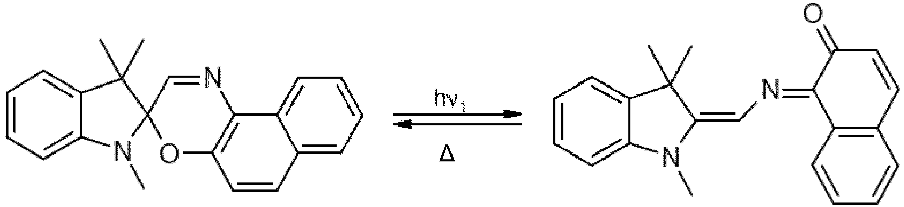

Die ungefärbten Verbindungen zeichnen sich durch einen geschlossenen Ring (linke Formel), die gefärbten Verbindungen durch einen offenen Ring aus (rechte Formel). Die erfindungsgemäßen Werkstoffe werden vorzugsweise in der ungefärbten Form vertrieben, und dementsprechend werden die photochromen Farbstoffe hier durch Verwendung der Formeln für die ungefärbten Verbindungen definiert. Es versteht sich jedoch von selbst, dass auch solche Werkstoffe, die die entsprechenden gefärbten Verbindungen enthalten, Gegenstand der Erfindung sind.

Erfindungsgemäß sind solche photochromen Farbstoffe bevorzugt, die mit Licht einer Wellenlänge λ₁ von 350 bis 800 nm, vorzugsweise 370 bis 490 nm, besonders bevorzugt 370 bis 470 nm und ganz besonders bevorzugt mit Licht einer Wellenlänge im Bereich von 415 bis 465 nm angeregt werden können. Dies ermöglicht die Verwendung von handelsüblichen dentalen Polymerisationslampen zur Anregung des Farbumschlags. Hierbei sind Farbstoffe besonders bevorzugt sind, die in nicht angeregten Zustand keine oder nur wenig Eigenfarbe aufweisen.

Es ist außerdem bevorzugt, dass die erfindungsgemäßen Dentalwerkstoffe im farbigen Zustand eine kontrastreiche Farbe im Vergleich zur Zahnhartsubstanz und zu ggf. vorhandenem Karies aufweisen. Daher sind Farbstoffe bevorzugt, die im angeregten Zustand rot, rosa, grün oder blau gefärbt sind, und sich gut von den bei Karies vorherrschenden Schwarz-, Braun- und Grautönen unterscheiden lassen. Der Farbton kann dabei durch die Auswahl und/oder Kombination geeigneter Farbstoffe eingestellt werden.

Der Kern der Kapseln ist flüssig, d.h. der Innenraum der Kapseln enthält eine Flüssigkeit, in der das oder die photochromen Farbmittel dispergiert oder gelöst sind. Die Flüssigkeit ist vorzugsweise ausgewählt aus organischen Lösungsmitteln, besonders bevorzugt aus Carbonsäureestern. Bevorzugt sind die Ester der Alkanole und Glycerinestern. Besonders bevorzugte Glycerinester sind Ester mit Fettsäuren, die 6 bis 18 Kohlenstoffatome, besonders bevorzugt 8 bis 16 und ganz besonders bevorzugt 10 bis 16 Kohlenstoffatome aufweisen, wobei Fettsäuren mit einer geraden Anzahl von Kohlenstoffatomen, wie in Miglyol (gemischte Decanoyl- und Octanoylglyceride) und insbesondere Glycerintricaprylat (Trioctanoin, CAS-Nr. 538-23-8), sowie Mischungen davon bevorzugt sind. Ganz besonders bevorzugt sind Miglyol und Trioctanoin.

Besonders bevorzugte Alkanolester sind Ester von C₁-C₈-, vorzugsweise C₁-C₆- und besonders bevorzugt C₁-C₃-Alkanolen mit Fettsäuren, die 6 bis 22 Kohlenstoffatome, besonders bevorzugt 8 bis 20 und ganz besonders bevorzugt 10 bis 18 Kohlenstoffatome aufweisen, wobei Fettsäuren mit einer geraden Anzahl von Kohlenstoffatomen, wie Caprinsäure, Laurinsäure, Myristinsäure, Palmitinsäure und insbesondere Stearinsäure besonders bevorzugt sind. Ganz besonders bevorzugt sind demgemäß die Ester von C₁-C₃-Alkanolen mit C₁₀-C₁₈-Fettsäuren sowie Mischungen davon. Ein besonders bevorzugter Ester ist Methylstearat.

Durch die Wahl der Flüssigkeit innerhalb der Kapselhülle kann die Kinetik der photochromen Reaktion beeinflusst werden. Relevante Parameter bei der Auswahl der Flüssigkeit sind z.B. Polarität, Hydrophobie und die Löslichkeit oder Dispergierbarkeit des Farbstoffs in der Flüssigkeit. In Kapseln mit einem flüssigen Kern lässt sich ein photochromes Verhalten erzielen, das dem des Farbmittels in Lösung entspricht. Der flüssige Kern bietet dem photochromen Farbstoff den nötigen Raum, der für den lichtinduzierten Übergang von der ungefärbten in die gefärbte Form und die thermische Rückreaktion erforderlich ist, und verhindert so Einschränkungen der photochromen Kinetik durch die umgebende Matrix.

Die Kapseln können auf an sich bekannte Weise mittels mechanischer, physikalischer, physikalisch-chemischer oder chemischer Verfahren hergestellt werden. Geeignete mechanische Verfahren sind z.B. die Sprühtrocknung oder das Beschichten in der Wirbelschicht. Geeignete physikalische bzw. physikalischchemische Verfahren sind die Koacervation sowie Lösungsmittelverdampfungs- und Lösungsmitteldiffusionsverfahren. Als chemische Verfahren eigenen sich z.B. die Grenzflächenpolymerisation, die Grenzflächenvernetzung und die In-situ-Polymerisation.

Geeignete Verfahren sind aus dem Stand der Technik bereits bekannt, wie beispielsweise R. J. Marathe, A. B. Chaudhari, R. K. Hedaoo, D. Sohn, V. R. Chaudhari, V. V. Gite, Urea formaldehyde (UF) microcapsules loaded with corrosion inhibitor for enhancing the anti-corrosive property of acrylic-based multifunctional PU coatings, RSC Advances, 5, 15539-15546, 2015;

Tatiya, P. D.; Gite, V. V. (2013). "Novel Polyurea Microcapsules Using Dendritic Functional Monomer: Synthesis, Characterization, and Its Use in Self-healing and Anticorrosive Polyurethane Coatings". Industrial & Engineering Chemistry Research. 52 (4): 1562-1570;

Hedaoo, R. K.; Tatiya, P. D.; Mahulikar, P. P.; Gite, V. V. (2013). "Fabrication of Dendritic 0G PAMAM-Based Novel Polyurea Microcapsules for Encapsulation of Herbicide and Release Rate from Polymer Shell in Different Environment". Design Monomers and Polymers. 2014 (2): 111-125;

Hedaoo, Rahul K., et al. "Fabrication of Core-Shell Novel Polyurea Microcapsules Using Isophorone Diisocyanate (IPDI) Trimer for Release System." International Journal of Polymeric Materials and Polymeric Biomaterials 63.7 (2014) 352-360;

Simon Benita (Herausgeber), Microencapsulation: Methods and Industrial Applications (Drugs and the Pharmaceutical Sciences), CRC Press, Taylor & Francis Group, Boca Raton 2006; Deutsche Apotheker Zeitung 16/2012, "Sprühtrocknung - so schnell kann Trocknen gehen! Von der Wirkstofflösung zum feinen Pulver", https://www.deutsche-apotheker-zeitung.de/daz-az/2012/daz-16-2012/spruehtrocknung-so-schnell-kann-trocknengehen.

Kapselmaterial, Wandstärke und Herstellungsverfahren werden vorzugsweise so gewählt, dass Kapseln mit einer flexiblen Hülle erhalten werden. Eine vorteilhafte Flexibilität lässt sich auf elektronenmikroskopischen Aufnahmen daran erkennen, dass sich die Ränder der angeschliffenen Verkapselung faltig und flexibel um das Farbmittel legen (siehe Beispiel 2 und Figuren 1 und 2). Die Kapseln haben vorzugsweise einen Durchmesser von 1 µm bis 10 µm, besonders bevorzugt von 2 µm bis 8 µm, wobei der Durchmesser mittels Ausmessen der Kapseln auf REM-Aufnahmen bestimmt wurde. Die Kapseln weisen vorzugsweise eine sphärische Form auf (siehe Figur 3).

Ein erfindungsgemäß besonders bevorzugt sind Kapseln, die den photochromen Farbstoff 6'-Piperidin-1,3,3,-trimethyl-spiro-[indolino-2,3'-[3H]naphth[2,1-b][1,4]oxazin] (CAS-Nr. 114747-45-4) enthalten, wobei die Kapseln eine Wand auf der Basis von Melamin-Formaldehyd-Harz (CAS-Nr. 13236-84-5) und einen flüssigen Kern aufweisen, der Trioctanoin (CAS-Nr. 538-23-8) enthält, und wobei der Farbstoff in dem flüssigen Kern enthalten ist.

Erfindungsgemäß geeignete, verkapselte photochrome Farbstoffe sind zum Teil kommerziell erhältlich.

Es wurde überraschend gefunden, dass verkapselte Farbmittel zur Herstellung von Dentalwerkstoffen geeignet sind. Es war nicht zu erwarten, dass flüssigkeitsgefüllte Kapseln die Herstellung von Werkstoffen erlauben, die nach der Härtung eine für dentale Anwendungen geeignete mechanische Festigkeit und Haltbarkeit aufweisen.

Die erfindungsgemäßen Dentalwerkstoffe ermöglichen eine gezielte Farbgebung bzw. Sichtbarmachung durch kurzzeitiges Bestrahlen mit Licht der Wellenlänge λ₁. Nach Beendung der Bestrahlung entfärben sich die Dentalwerkstoffe wieder. Dieser Vorgang läuft mit hoher Effizienz ab, selbst nach der Härtung der Werkstoffe durch radikalische Polymerisation.

Bei der Verwendung unverkapselter Farbstoffe wurde demgegenüber eine deutliche Abnahme der Farbumschlagsdauer nach der Polymerisation beobachtet, d.h. der Farbumschlag war nur für einen kurzen, praktisch nicht ausreichenden Zeitraum zu erkennen und häufig war kein Farbumschlag mehr möglich. In vielen Fällen wurde eine nicht reversible Verfärbung der Werkstoffe beobachtet.

Es ist bevorzugt, dass sich die erfindungsgemäßen Dentalwerkstoffe thermisch bedingt entfärben, vorzugsweise innerhalb einer Zeit von weniger als 30 Minuten, besonders bevorzugt weniger als 5 Minuten, ganz besonders bevorzugt weniger als 1,5 Minuten und am meisten bevorzugt weniger als 1,0 Minuten nach Beendigung der Anregung. Erfindungsgemäß besonders bevorzugte Werkstoffe entfärben sich innerhalb von 10 bis 90 Sekunden, vorzugsweise 10 bis 60 Sekunden vollständig.

Der Dauer des Farbumschlags ist für die Sichtbarmachung von Fissurenversiegelungen, zur Randkontrolle dentalen Restaurationen oder zur Kontrolle des Retentionserhalts ausreichend, und sie ermöglicht dem Zahnarzt eine abschließende Begutachtung der Färbung der Restauration innerhalb einer Behandlungssitzung.

Die Verkapselung der Farbmittel hat den weiteren Vorteil, dass sich die Farbmittel besser mit den übrigen Bestandteilen mischen lassen, die zur Herstellung der Dentalwerkstoffe erforderlich sind. Die mit Farbstoff beladenen Kapseln können ohne Vordispergierung direkt mit den übrigen Komponenten gemischt werden, und es wird eine höhere Homogenität erzielt. Eine Agglomeration der Farbstoffe in der Zusammensetzung wurde nicht beobachtet. Die Kapseln sind mit unterschiedlichsten Matrixmaterialien kompatibel, und es ist nicht erforderlich, die Polymermatrix beispielsweise durch die Anpassung der Glasübergangstemperatur T_{g} so auf die Farbstoffe abzustimmen, dass der angestrebte photochrome Effekt erreicht wird.

Außerdem gewährleistet die Verkapselung eine hohe Stabilität der Farbstoffe, wodurch der Farbumschlag wiederholt über einen längeren Zeitraum herbeigeführt werden kann, was im Hinblick auf spätere Nachuntersuchungen vorteilhaft ist.

Darüber hinaus verhindert die Verkapselung, dass der photochrome Farbstoff aus dem Dentalwerkstoff ausgewaschen wird.

Die erfindungsgemäßen Dentalwerkstoffe enthalten neben verkapselten photochromen Farbstoffen vorzugsweise zusätzlich mindestens ein radikalisch polymerisierbares Monomer, insbesondere ein mono- und/oder polyfunktionelles (Meth)acrylsäurederivat oder eine Mischung davon. Besonders bevorzugt sind Werkstoffe, die als radikalisch polymerisierbares Monomer mindestens ein multifunktionelles (Meth)acrylat oder eine Mischungen von mono- und multifunktionellen (Meth)acrylaten enthalten. Unter monofunktionellen (Meth)acrylaten werden Verbindungen mit einer, unter polyfunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Werkstoffe, die als radikalisch polymerisierbares Monomer mono- und multifunktionelle (Meth)acrylate enthalten, eignen sich besonders als Dentalwerkstoffe. In allen Fällen sind Methacrylate bevorzugt. Es wurde gefunden, dass die verkapselten photochromen Farbstoffe mit den hier genannten Monomeren gut verträglich sind und homogene Mischungen bilden, die bei der Polymerisation Materialien mit mechanischen Eigenschaften ergeben, wie sie für Dentalwerkstoffe erwünscht sind.

Bevorzugte mono- oder polyfunktionelle Methacrylate sind Methyl-, Ethyl-, Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)), Bis(methacryloyloxymethyl)tricyclo[5.2.1.]decan (TCDMA), ethoxy- oder propoxyliertes Bisphenol-A-di(meth)-acrylat, wie z.B. das Bisphenol-A-dimethacrylat 2-[4-(3-Methacryloyloxyethoxyethyl)phenyl]-2-[4-(3-methacryloyloxyethyl)-phenyl]-propan) (SR-348c) mit 3 Ethoxygruppen oder 2,2-Bis[4-(2-(meth)acryloxypropoxy)phenyl]propan, Di-, Tri- oder Tetraethylenglycoldi(meth)acrylat, Trimethylolpropantri(meth)-acrylat, Pentaerythrittetra(meth)acrylat, sowie Glycerin-di(meth)acrylat, 1,4-Butandioldi(meth)acrylat, 1,10-Decandiol-di(meth)acrylat, 1,12-Dodecandioldi(meth)acrylat oder Glycerintrimethacrylat (GTMA).

Außerdem sind nicht acide Ester der Phosphorsäure mit Hydroxymethacrylaten als radikalisch polymerisierbare Monomere bevorzugt. Besonders bevorzugt sind die Ester der ω-Hydroxy-C₂-C₁₀-alkylmethacrylate. Ganz besonders bevorzugte Beispiele sind Tris[2-(methacryloyloxy)ethyl]phosphat, 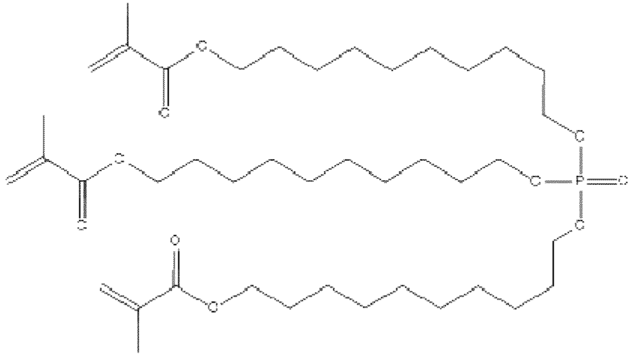 und

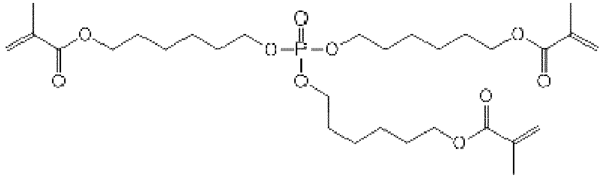

Weiter bevorzugt sind N-mono- oder -disubstituierte Acrylamide, wie z.B. N-Ethylacrylamid, N,N-Dimethylacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxyethyl)ethacrylamid sowie N-Vinylpyrrolidon. Diese Monomere zeichnen sich durch eine geringe Viskosität und eine hohe Hydrolysestabilität aus und eignen sich besonders als Verdünnermonomere.

Ebenfalls bevorzugt sind vernetzende Pyrrolidone, wie z.B. 1,6-Bis(3-vinyl-2-pyrrolidonyl)-hexan oder kommerziell zugängliche Bisacrylamide, wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Diese Monomere zeichnen sich durch eine hohe Hydrolysestabilität aus und eignen sich besonders als Vernetzermonomere.

Die erfindungsgemäßen Werkstoffe enthalten vorzugsweise keine säuregruppenhaltigen Monomere, wie Carbonsäuren, Phosphonsäuren oder Sulfonsäuren.

Die erfindungsgemäßen Dentalwerkstoffe enthalten vorzugsweise auch einen Initiator für die radikalische Polymerisation, besonders bevorzugt einen Photoinitiator.

Zur Initiierung der radikalischen Photopolymerisation sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil bevorzugt. Besonders bevorzugt werden Campherchinon und 2,2-Dimethoxy-2-phenyl-acetophenon und besonders bevorzugt α-Diketone in Kombination mit Aminen als Reduktionsmittel verwendet, wie z.B. 4-(Dimethylamino)benzoesäureethylester, N,N-Dimethylaminoethylmethacrylat, N,N-Dimethyl-sym.-xylidin oder Triethanolamin. Besonders geeignet sind auch Norrish-Typ-I-Photoinitiatoren, vor allem Acyl- oder Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich vorteilhaft auch Mischungen der verschiedenen Photoinitiatoren einsetzen, wie z.B. Dibenzoyldiethylgermanium in Kombination mit Campherchinon und 4-Dimethylaminobenzoesäureethylester.

Als Initiatoren für eine bei Raumtemperatur durchgeführte Polymerisation werden vorzugsweise Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden bzw. Hydroperoxiden und solchen Reduktionsmitteln, wie z.B. Ascorbinsäure, Barbiturate, Thioharnstoffe oder Sulfinsäuren, besonders geeignet.

Weiterhin enthalten die erfindungsgemäßen Dentalwerkstoffe vorzugsweise auch mindestens einen organischen oder besonders bevorzugt anorganischen partikulären Füllstoff. Bevorzugt sind Füllstoffe auf der Basis von Oxiden, wie SiO₂, ZrO₂ und TiO₂ bzw. Mischoxiden aus SiO₂, ZrO₂, ZnO und/oder TiO₂, nanopartikuläre oder mikrofeine Füllstoffe, wie pyrogene Kieselsäure oder Fällungskieselsäure (gewichtsmittlere Partikelgröße von 10-1000 nm) sowie Minifüllstoffe, wie Quarz-, Glaskeramik- oder röntgenopake Glaspulver von z.B. Barium- oder Strontiumaluminiumsilikatgläsern (gewichtsmittlere Partikelgröße von 0,2-10 µm). Weitere bevorzugte Füllstoffe sind röntgenopake Füllstoffe, wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid (gewichtsmittlere Partikelgröße von 10-1000 nm).

Zur Verbesserung des Verbundes zwischen den Füllstoffpartikeln und der vernetzten Polymerisationsmatrix können SiO₂-basierende Füllstoffe mit methacrylat-funktionalisierten Silanen, wie z.B. 3-Methacryloyloxypropyltrimethoxysilan, oberflächenmodifiziert werden. Zur Oberflächenmodifizierung von nichtsilikatischen Füllstoffen, z.B. von ZrO₂ oder TiO₂, können auch funktionalisierte saure Phosphate, wie z.B. 10-Methacryloyloxydihydrogenphospaphat eingesetzt werden.

Die erfindungsgemäßen Dentalwerkstoffe können in Abhängigkeit vom gewünschten Einsatzzweck vorzugsweise auch ein Lösungsmittel enthalten, insbesondere Wasser, Ethanol oder eine Mischung davon.

Außerdem können die erfindungsgemäßen Werkstoffe weitere Additive enthalten, vor allem Stabilisatoren, wie z.B. Polymerisationsstabilisatoren, Aromastoffe, Farbmittel, mikrobiozide Wirkstoffe, fluoridionenabgebende Additive, optische Aufheller, Fluoreszenzmittel, Weichmacher, und/oder UV-Absorber.

Dabei sind erfindungsgemäß solche Dentalwerkstoffe bevorzugt, die die folgenden Komponenten enthalten:
a) 0,0001 bis 5 Gew.-%, bevorzugt 0,001 bis 3,0 Gew.-% und besonders bevorzugt 0,1 bis 1,0 Gew.-% mindestens eines verkapselten photochromen Farbmittels,
b) 0,01 bis 10 Gew.-%, besonders bevorzugt 0,1 bis 3,0 Gew.-% mindestens eines Initiators für die radikalische Polymerisation, und
c) 5 bis 85 Gew.-%, bevorzugt 10 bis 60 Gew.-% und besonders bevorzugt 15 bis 50 Gew.-% mindestens eines radikalisch polymerisierbaren Monomers.

Außerdem können die erfindungsgemäßen Werkstoffe optional
d) 2,50 bis 80 Gew.-% Füllstoff(e) und/oder
e) 2,50 bis 70 Gew.-% Lösungsmittel enthalten.

Alle Mengenangaben beziehen sich, wenn nicht anders angegeben, auf die Gesamtmasse des Dentalwerkstoffs.

Der Gehalt an photochromen Farbstoff richtet sich primär nach dem gewünschten Farbeffekt.

Dentalwerkstoffe zur Verwendung als Fissurenversiegler enthalten bevorzugt die folgenden Komponenten:
a) 0,0001 bis 5 Gew.-%, bevorzugt 0,001 bis 3,0 Gew.-% und besonders bevorzugt 0,1 bis 1,0 Gew.-% mindestens eines verkapselten photochromen Farbmittels,
b) 0,01 bis 10 Gew.-%, besonders bevorzugt 0,1 bis 3,0 Gew.-% mindestens eines Initiators, und
c) 5 bis 85 Gew.-%, bevorzugt 10 bis 60 Gew.-% und besonders bevorzugt 15 bis 50 Gew.-% mindestens eines Monomers.

Außerdem können die erfindungsgemäßen Fissurenversiegler optional
d) 2,50 bis 80 Gew.-% Füllstoff(e) und/oder
e) 2,50 bis 70 Gew.-% Lösungsmittel enthalten.

Besonders bevorzugt sind jeweils solche Werkstoffe, die aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind.

Die erfindungsgemäßen Werkstoffe eignen sich besonders als Dentalwerkstoffe, insbesondere als dentale Fissurenversiegler, dentaler Zement, dentales Füllungskomposit oder dentales Verblendmaterial. Sie zeichnen sich durch reversible photochrome Eigenschaften aus.

Die Dentalwerkstoffe eignen sich primär zur intraoralen Anwendung durch den Zahnarzt zur prophylaktischen Versiegelung von Fissuren, aber auch zur Restauration geschädigter Zähne, d.h. zur therapeutischen Anwendung, z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken.

Die Erfindung wird im Folgenden anhand von Figuren und Ausführungsbeispielen näher erläutert.

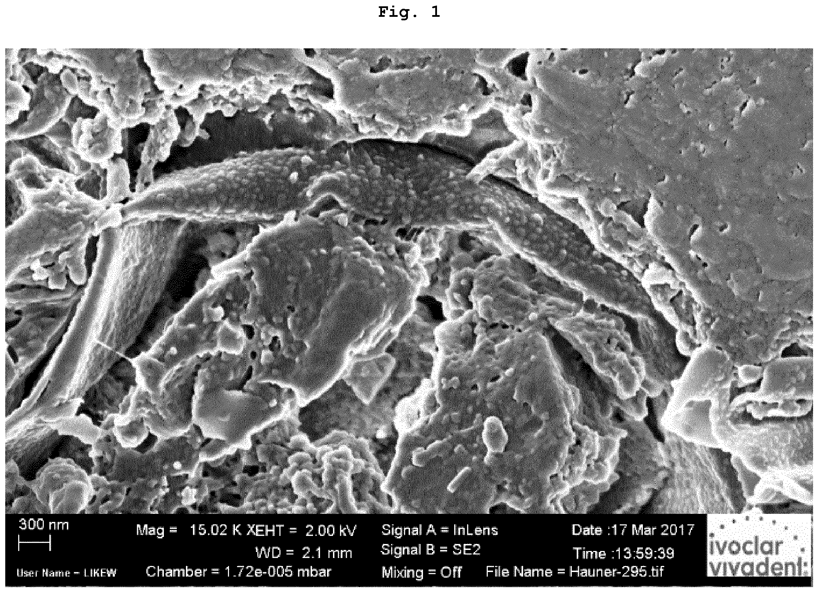

Fig. 1 zeigt eine REM-Aufnahme vom Rand der Hülle einer angeschliffenen Kapsel an der Oberfläche eines Plättchens aus einem erfindungsgemäßen Fissurenversiegler nach der Härtung des Plättchens und nach dem Anschleifen der Plättchenoberfläche. Die Ränder der Kapselwand legen sich faltig und flexibel um den photochromen Farbstoff.

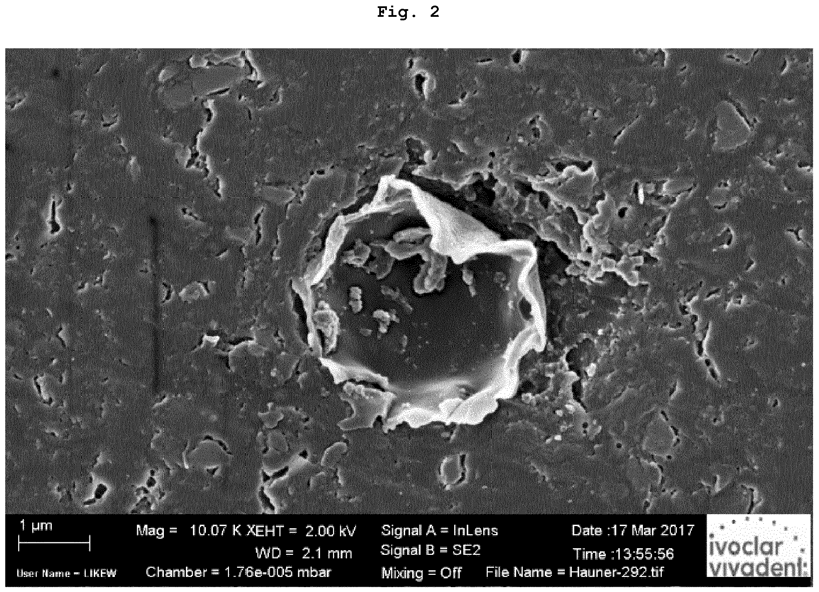

Fig. 2 zeigt die REM-Aufnahme einer leeren Kapselhülle an der Oberfläche des Plättchens. Die Ränder der Kapselwand legen sich faltig und flexibel um den Rand des gehärteten Fissurenversieglers.

Die Aufnahmen zeigen, dass die Kapselwand flexibel ist.

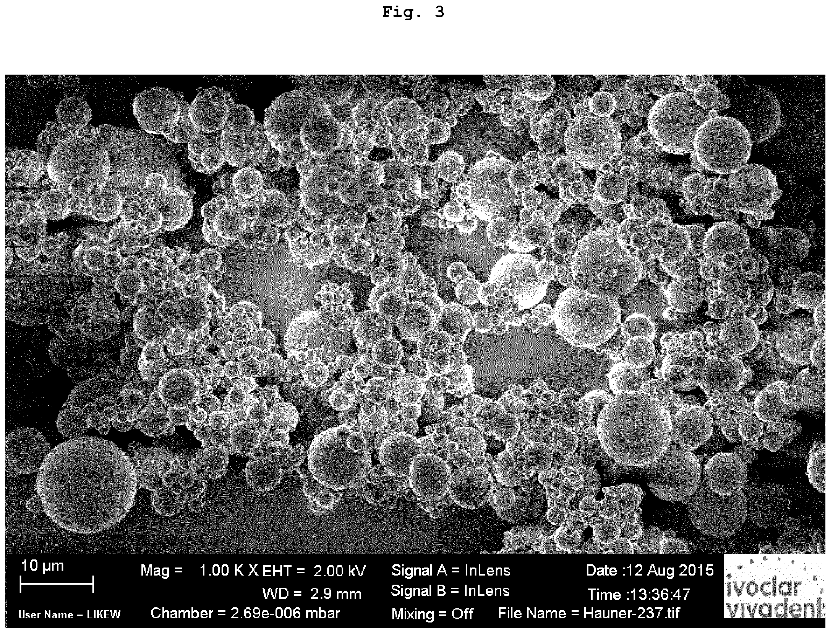

Fig. 3 zeigt die REM-Aufnahme einer Ansammlung erfindungsgemäßer Kapseln. Die Kapseln haben eine sphärische Form und weisen einen Durchmesser von ca. 1 µm bis ca. 10 µm auf.

### Ausführungsbeispiele

### Beispiel 1:

### Herstellung eines photochromen Fissurenversieglers

Die in Tabelle 1 beschriebene Zusammensetzung (Anteile in Gew.-%) wurde hergestellt, indem zunächst die Initiatoren in der Monomermischung gelöst wurden und danach die erhaltene Lösung mit den Füllstoffen, Farbstoffen und den übrigen Komponenten vermischt wurde. Abschließend wurde die Zusammensetzung mit einem Dreiwalzenstuhl homogenisiert.

Von den Zusammensetzungen wurden runde Prüfkörper (Durchmesser 10 mm, Höhe: 1 mm) präpariert, die 2 x 1 Minute mit einer dentalen Lichtquelle (Spectramat®, Ivoclar Vivadent AG) ausgehärtet wurden, wobei hier bereits eine vorübergehende Verfärbung auftrat. Es ergaben sich weiße Prüfkörper, die sich beim Bestrahlen (2 s) mit einem LED-basierten Lichtpolymerisationsgerät (λ₁ = 415-465 nm) pink verfärbten. Nach Beendigung der Bestrahlung entfärbten sich die Prüfkörper bei Raumtemperatur innerhalb von 30 s bis 60 s. Das wechselseitige Verfärben und Entfärben ließ sich auch noch nach Wasserlagerung für 76 Wochen ohne Einschränkungen wiederholen.

**Tabelle 1: Zusammensetzung des Werkstoffs**

| **Funktion** | **Komponente** | **Menge [Gew - %]** |
|---|---|---|
| Monomer | RM 3¹⁾ | 40,86 |
| | Viscoat-3PMA²⁾ | 24,62 |
| | V380³⁾ | 11,48 |
| Initiator | Chivacure EPD⁴⁾ | 0,39 |
| | Campherchinon | 0,23 |
| | BHT Purum⁵⁾ (Stabilisator) | 0,02 |
| | TEMPO⁶⁾ (Stabilisator) | 0,01 |
| Füllstoff | HDK 2000⁷⁾ | 4,95 |
| | Glasfüller, 7 µm sil 6,0%⁸⁾ | 14,85 |
| Farbstoff | Edaplan 516⁹⁾ | 0,99 |
| | Farbpigment Aeroxide TiO2P25¹⁰⁾ | 0,59 |
| | photochrome pigment red¹¹⁾ | 1,00 |

| | | |
|---|---|---|
| ¹⁾ Urethandimethacrylat ²⁾ Tris[2-(methacryloyloxy)ethyl]phosphat ³⁾ Aromatisches und aliphatisches Urethandimethacrylat ⁴⁾ p-Dimethylaminobenzoesäureethylester ⁵⁾ Butylhydroxytoluol ⁶⁾ 2,2,6,6-Tetramethylpiperidinyloxyl ⁷⁾ pyrogene Kieselsäure (Wacker), spezifische Oberfläche (BET) ca. 200 m²/g ⁸⁾ silanisierter Glasfüller G018-090 UF0 ⁹⁾ Polycarbonsäuresalz (Dispergiermittel für Pigmente) ¹⁰⁾ Titandioxid (Evonik) mit einer spezifischen Oberfläche (BET) von ca. 50 m²/g ¹¹⁾ Kapseln mit einer Hülle aus Melamin-Formaldehyd-Harz, die den Farbstoff 6'-Piperidin-1,3,3,-trimethyl-spiro[indolino-2,3'-[3H]naphth[2,1-b][1,4]oxazin] (CAS-Nr. 114747-45-4) und Methylstearat enthalten | | |

Die Biegefestigkeit des Fissurenversieglers mit dem verkapselten photochromen Farbstoff war erstaunlicherweise mit der Biegefestigkeit eines Fissurenversieglers ohne verkapselten Farbstoff vergleichbar. Für den Fissurenversiegler mit verkapseltem photochromem Farbstoff wurde eine Biegefestigkeit von 79 ± 15 MPa und für den Fissurenversiegler ohne verkapselten photochromen Farbstoff eine Biegefestigkeit von 71 ± 13 MPa gemessen (jeweils gemäß ISO 4940).

### Beispiel 2:

### Elektronenmikroskopische Untersuchung des Fissurenversieglers

Es wurde ein Plättchen aus dem in Beispiel 1 beschriebenen Fissurenversiegler hergestellt. Die Herstellung des Plättchens erfolgte analog zu Beispiel 1. Nach der Härtung wurde die Oberfläche des Plättchens poliert, im Ultraschallbad gereinigt und dann elektronenmikroskopisch untersucht. Fig. 1 zeigt eine REM-Aufnahme vom Rand der Hülle einer angeschliffenen Kapsel an der Oberfläche des polierten Plättchens. Fig. 2 zeigt die REM-Aufnahme einer leeren Kapselhülle an der Oberfläche des Plättchens. Es ist zu erkennen, dass sich die Ränder der Kapsel faltig und flexibel um den photochromen Farbstoff (Fig. 1) bzw. den Rand des gehärteten Fissurenversieglers (Fig. 2) legen. Die Abbildungen zeigen damit, dass die Kapselwand flexibel ist. Fig. 3 zeigt die photochromen Kapseln ohne Matrix auf einem REM-Träger.

### Beispiel 3:

### Zweikomponentiger Dentalwerkstoff

Durch Mischen der in den Tabelle 2 und 3 aufgeführten Komponenten wurden eine Katalysator- und eine Basenpaste hergestellt. Zur Härtung wurden die Pasten im Verhältnis von 1:1 miteinander gemischt. Nach der Härtung war der Werkstoff reversibel photochrom. Das Beispiel zeigt, dass auch photochrome Werkstoffe mit hohem Füllstoffgehalte hergestellt werden können. Die photochromen Kapseln werden trotz des hohen Füllstoffanteils beim Mischen der Komponenten nicht zerstört. Die Pasten eignen sich besonders zur Verarbeitung in einer Doppelkammerspritze.

**Tabelle 2: Zusammensetzung der Katalysatorpaste**

| **Funktion** | **Komponente** | **Menge [Gew.-%]** |
|---|---|---|
| Monomer | RM 3¹⁾ | 22,57 |
| | Viscoat-3PMA²⁾ | 10, 40 |
| Initiator | Cumolhydroperoxid | 1,49 |
| Stabilisator | BHT⁵⁾ | 0,07 |
| Füllstoff | HDK 2000⁷⁾ | 2,08 |
| | Barium Glasfüller 1,0 µm¹⁴⁾ | 6,44 |
| | Silica -Zirconia -Mixed-Oxide¹⁵) | 44,95 |
| | YbF₃ | 11,00 |
| Farbstoff | photochrome pigment red¹¹⁾ | 1,00 |

| | | |
|---|---|---|
| ¹⁾⁻¹¹⁾ wie Tabelle 1 ¹⁴⁾ silanisierter Glasfüller (CAS-Nr. 65997-18-4) ¹⁵⁾ Sphärosil | | |

**Tabelle 3: Zusammensetzung der Basenpaste**

| **Funktion** | **Komponente** | **Menge [Gew. -%]** |
|---|---|---|
| Monomer | RM 3¹⁾ | 23,40 |
| | Viscoat-3PMA²⁾ | 10,68 |
| Initiator | Photoinitiator K69¹²⁾ | 0,03 |
| | Hexanoylthioharnstoff¹³⁾ | 0,52 |
| | Kupferacetylacetonat | 0,01 |
| Stabilisator | BHT⁵⁾ | 0,02 |
| | TEMPO⁶⁾ | 0,001 |
| Füllstoff | HDK 2000⁷⁾ | 2,08 |
| | Barium Glasfüller 1,0 µm¹⁴⁾ | 6,44 |
| | YbF₃ | 10,89 |
| | Silica-Zirconia-Mixed-Oxide¹⁵⁾ | 44,95 |
| Farbstoff | photochrome pigment red¹¹⁾ | 1,00 |

| | | |
|---|---|---|
| ¹⁾⁻¹¹⁾ wie Tabelle 1 ¹²⁾ Ivocerin (CAS-Nr: 1469766-31-1) ¹³⁾ K107 (CAS-Nr. 41510-13-8) ¹⁴⁾ silanisierter Glasfüller (CAS-Nr. 65997-18-4) ¹⁵⁾ Sphärosil | | |

## Patentansprüche

1. Radikalisch polymerisierbarer Dentalwerkstoff, der mindestens ein photochromes Farbmittel enthält, **dadurch gekennzeichnet, dass** das Farbmittel in eine Polymerkapsel eingeschlossen ist, die eine äußere Kapselwand und einen inneren Kern aufweist, wobei die Kapselwand durch ein organisches Polymer gebildet wird und der Kern flüssig ist und wobei der Kern das Farbmittel in gelöster oder dispergierter Form enthält.

2. Dentalwerkstoff nach Anspruch 1, der als Farbmittel mindestens einen photochromen organischen Farbstoff oder ein photochromes organisches Pigment enthält.

3. Dentalwerkstoff nach Anspruch 1 oder 2, bei dem die Kapselwand durch ein organisches Polymer auf der Basis von Melamin-Formaldehyd-Harz, Polyester, Polyamid, Polyurethan, Polysaccharid und/oder Harnstoff-Formaldehyd-Harz gebildet wird.

4. Dentalwerkstoff nach Anspruch 3, wobei die Kapselwand durch Melamin-Formaldehyd-Harz auf der Basis von N-(4,6-Diamino-1,3,5-triazin-2-yl)formamid (CAS-Nr. 13236-84-5) gebildet wird.

5. Dentalwerkstoff nach einem der vorhergehenden Ansprüche, der ein photochromes Farbmittel enthält, dessen Farbumschlag mit Licht einer Wellenlänge von 350 bis 800 nm, vorzugsweise mit Licht einer Wellenlänge von 370 bis 490 nm angeregt werden kann.

6. Dentalwerkstoff nach einem der vorhergehenden Ansprüche, bei dem das photochrome Farbmittel ein photochromer organischer Farbstoff ist, der aus Spirooxazinen, Spiropyranen, Azobenzolverbindungen, Stilbenen, Pentadienyl-Anionen und Kombinationen davon ausgewählt ist.

7. Dentalwerkstoff nach Anspruch 6, bei dem der photochrome organische Farbstoff ein Spirooxazin, vorzugsweise ein Spirooxazin mit dem Grundkörper 1,3,3-Trimethyl-spiro-[indolino-2,3'-[3H]napht[2,1-b][1,4]oxazin (NISO) und besonders bevorzugt 6'-Piperidin-1,3,3,-trimethyl-spiro-[indolino-2,3'-[3H]naphth[2,1-b][1,4]oxazin] ist.

8. Dentalwerkstoff nach einem der vorhergehenden Ansprüche, bei dem der Kern der Kapseln eine Flüssigkeit enthält, die aus organischen Lösungsmitteln, Carbonsäureestern, Glycerinestern, und Mischungen davon, vorzugsweise aus Miglyol, Trioctanoin und/oder Methylstearat ausgewählt ist.

9. Dentalwerkstoff nach einem der vorhergehenden Ansprüche, der zusätzlich mindestens ein radikalisch polymerisierbares Monomer, vorzugsweise ein mono- oder polyfunktionelles (Meth)acrylsäurederivat, und mindestens einen Initiator für die radikalische Polymerisation enthält, vorzugsweise einen Photoinitiator.

10. Dentalwerkstoff nach Anspruche 9, der als radikalisch polmymerisierbares Monomer mindestens ein mono- und/oder polyfunktionelles (Meth)acrylsäurederivat, mindestens einen nicht aciden Ester der Phosphorsäure mit einem Hydroxymethacrylat oder eine Mischung davon enthält.

11. Dentalwerkstoff nach einem der vorhergehenden Ansprüche, der zusätzlich mindestens einen partikulären Füllstoff enthält.

12. Dentalwerkstoff nach einem der vorhergehenden Ansprüche, der
a) 0,0001 bis 5 Gew.-%, bevorzugt 0,001 bis 3,0 Gew.-% und besonders bevorzugt 0,1 bis 1,0 Gew.-% mindestens eines verkapselten photochromen Farbmittels,
b) 0,01 bis 10 Gew.-%, besonders bevorzugt 0,1 bis 3,0 Gew.-% mindestens eines Initiators, und ggf.
c) 5 bis 85 Gew.-%, bevorzugt 10 bis 60 Gew.-% und besonders bevorzugt 15 bis 50 Gew.-% mindestens eines Monomers, und ggf.
d) 2,5 bis 80 Gew.-% Füllstoff(e) und ggf.
e) 2,5 bis 70 Gew.-% Lösungsmittel,
enthält, jeweils bezogen auf die Gesamtmasse des Dentalwerkstoffs.

13. Dentalwerkstoff nach Anspruch 12 zur Verwendung als Fissurenversiegler, der
a) 0,0001 bis 5 Gew.-%, bevorzugt 0,001 bis 3,0 Gew.-% und besonders bevorzugt 0,1 bis 1,0 Gew.-% mindestens eines verkapselten photochromen Farbmittels,
b) 0,01 bis 10 Gew.-%, besonders bevorzugt 0,1 bis 3,0 Gew.-% mindestens eines Initiators, und ggf.
c) 5 bis 85 Gew.-%, bevorzugt 10 bis 60 Gew.-% und besonders bevorzugt 15 bis 50 Gew.-% mindestens eines Monomers, und ggf.
d) 2,5 bis 80 Gew.-% Füllstoff(e) und ggf.
e) 2,5 bis 70 Gew.-% Lösungsmittel
enthält, jeweils bezogen auf die Gesamtmasse des Dentalwerkstoffs.

14. Dentalwerkstoff nach einem der Ansprüche 1 bis 13 zur therapeutischen Verwendung zur Versiegelung von Zahnfissuren, zur Restauration geschädigter Zähne, als dentaler Zement, dentales Füllungskomposit oder Verblendmaterial.

15. Verwendung eines verkapselten photochromen Farbmittels wie in einem der Ansprüche 1 bis 8 definiert zur Herstellung eines photochromen Dentalwerkstoffs.

16. Verwendung eines Dentalwerkstoffs nach einem der Ansprüche 1 bis 13 zur extraoralen Herstellung oder Reparatur von Dentalrestaurationen, wie Prothesen, künstlichen Zähnen, Inlays, Onlays, Kronen oder Brücken.

## Claims

1. Radically polymerisable dental material, comprising at least one photochromic colourant, **characterised in that** the colourant is encapsulated, wherein the colourant is encased in a polymer capsule that has an outer capsule wall and an inner core, wherein the capsule wall is formed by an organic polymer and the core is liquid, and wherein the core comprises the colourant in a dissolved or dispersed form.

2. Dental material according to claim 1, comprising at least one photochromic organic colouring agent or a photochromic organic pigment as the colourant

3. Dental material according to claim 1 or 2, wherein the capsule wall is formed by an organic polymer based on melamine-formaldehyde resin, polyester, polyamide, polyurethane, polysaccharide and/or urea-formaldehyde resin.

4. Dental material according to claim 3, wherein the capsule wall is formed by melamine-formaldehyde resin based on *N*-(4,6-diamino-1,3,5-triazin-2-yl)formamide (CAS No. 13236-84-5).

5. Dental material according to one of the preceding claims, comprising a photochromic colourant, the colour change of which can be induced with light having a wavelength of from 350 to 800 nm, preferably with light having a wavelength of from 370 to 490 nm.

6. Dental material according to one of the preceding claims, wherein the photochromic colourant is a photochromic organic colouring agent, selected from spirooxazines, spiropyrans, azobenzene compounds, stilbenes, pentadienyl anions and combinations thereof.

7. Dental material according to claim 6, wherein the photochromic organic colouring agent is a spirooxazine, preferably a spirooxazine based on the parent structure 1,3,3-trimethylspiro[indoline-2,3'-[3*H*]naphtho[2,1-*b*][1,4]oxazine (NISO), and particularly preferably 6'-piperidine-1,3,3,-trimethylspiro[indoline-2,3'-[3*H*]naphtho[2,1-*b*][1,4]oxazine].

8. Dental material according to one of the preceding claims, wherein the core of the capsules comprises a liquid, selected from organic solvents, carboxylic acid esters, glycerol esters and mixtures thereof, preferably from Miglyol, trioctanoin and/or methyl stearate.

9. Dental material according to one of the preceding claims, additionally comprising at least one radically polymerisable monomer, preferably a mono- or polyfunctional (meth)acrylic acid derivative, and at least one initiator for the radical polymerisation, preferably a photoinitiator.

10. Dental material according to claim 9, comprising, as the radically polymerisable monomer, at least one mono- and/or polyfunctional (meth)acrylic acid derivative, at least one non-acidic ester of phosphoric acid with a hydroxymethacrylate or a mixture thereof.

11. Dental material according to one of the preceding claims, additionally comprising at least one particulate filler.

12. Dental material according to one of the preceding claims, comprising
a) 0.0001 to 5 wt %, preferably 0.001 to 3.0 wt % and particularly preferably 0.1 to 1.0 wt % of at least one encapsulated photochromic colourant,
b) 0.01 to 10 wt %, particularly preferably 0.1 to 3.0 wt % of at least one initiator, and optionally
c) 5 to 85 wt %, preferably 10 to 60 wt % and particularly preferably 15 to 50 wt % of at least one monomer, and optionally
d) 2.5 to 80 wt % filler(s) and optionally
e) 2.5 to 70 wt % solvent,
in each case relative to the total mass of the dental material.

13. Dental material according to claim 12 for use as a fissure sealant, comprising
a) 0.0001 to 5 wt %, preferably 0.001 to 3.0 wt % and particularly preferably 0.1 to 1.0 wt % of at least one encapsulated photochromic colourant,
b) 0.01 to 10 wt %, particularly preferably 0.1 to 3.0 wt % of at least one initiator, and optionally
c) 5 to 85 wt %, preferably 10 to 60 wt % and particularly preferably 15 to 50 wt % of at least one monomer, and optionally
d) 2.5 to 80 wt % filler(s) and optionally
e) 2.5 to 70 wt % solvent,
in each case relative to the total mass of the dental material.

14. Dental material according to one of claims 1 to 13 for therapeutic use for sealing fissures in teeth, for the restoration of damaged teeth, as a dental cement, dental filling composite or veneering material.

15. Use of an encapsulated photochromic colourant, as defined in one of claims 1 to 8, for the production of a photochromic dental material.

16. Use of a dental material according to one of claims 1 to 13 for the extraoral production or repair of dental restorations, such as prostheses, artificial teeth, inlays, onlays, crowns or bridges.

## Revendications

1. Matériau dentaire polymérisable par voie radicalaire, contenant au moins un agent colorant photochrome, **caractérisé en ce que** cet agent colorant est enfermé dans une capsule de polymère, qui comporte une paroi externe de capsule et un noyau interne et dans laquelle la paroi de capsule est constituée d'un polymère organique et le noyau, liquide, contient l'agent colorant à l'état dissous ou dispersé.

2. Matériau dentaire conforme à la revendication 1, qui contient en tant qu'agent colorant, au moins un colorant organique photochrome ou un pigment organique photochrome.

3. Matériau dentaire conforme à la revendication 1 ou 2, dans lequel la paroi de capsule est constituée d'un polymère organique à base de résine mélamine-formaldéhyde, de polyester, de polyamide, de polyuréthane, de polysaccharide et/ou de résine urée-formaldéhyde.

4. Matériau dentaire conforme à la revendication 3, dans lequel la paroi de capsule est constituée d'une résine mélamine-formaldéhyde à base de N-(4,6-diamino-1,3,5-triazin-2-yl)-formamide (N° CAS : 13236-84-5).

5. Matériau dentaire conforme à l'une des revendications précédentes, qui contient un agent colorant photochrome dont on peut activer le changement de couleur au moyen d'un rayonnement dont la longueur d'onde vaut de 350 à 800 nm, et de préférence, au moyen d'un rayonnement dont la longueur d'onde vaut de 370 à 490 nm.

6. Matériau dentaire conforme à l'une des revendications précédentes, dans lequel l'agent colorant photochrome est un colorant photochrome organique qui est choisi parmi les spiro-oxazines, spiro-pyranes, composés de type azo-benzène, stilbènes et anions pentadiényle, ainsi que leurs combinaisons.

7. Matériau dentaire conforme à la revendication 6, dans lequel le colorant organique photochrome organique est une spiro-oxazine, et de préférence une spiro-oxazine ayant comme squelette de base celui de la 1,3,3-triméthyl-spiro[indolino-2,3'-[3H]napht[2,1-b]-1,4-oxazine] (NISO), et en particulier, la 6'-pipéridino-1,3,3-triméthyl-spiro[indolino-2,3'-[3H]napht[2,1-b]-1,4-oxazine].

8. Matériau dentaire conforme à l'une des revendications précédentes, dans lequel le noyau des capsules contient un liquide qui est choisi parmi les solvants organiques, esters carboxylates, esters de glycérol et leurs mélanges, et de préférence, parmi les miglyols, la tri-octanoïne et/ou le stéarate de méthyle.

9. Matériau dentaire conforme à l'une des revendications précédentes, qui contient en outre au moins un monomère polymérisable par voie radicalaire, de préférence un dérivé monofonctionnel ou polyfonctionnel d'acide acrylique ou méthacrylique, et au moins un amorceur de polymérisation radicalaire, de préférence un photo-amorceur.

10. Matériau dentaire conforme à la revendication 9, qui contient, en tant que monomère polymérisable par voie radicalaire, au moins un dérivé monofonctionnel et/ou polyfonctionnel d'acide acrylique ou méthacrylique, au moins un ester non acide d'acide phosphorique et d'un hydroxy-méthacrylate, ou un mélange de tels composés.

11. Matériau dentaire conforme à l'une des revendications précédentes, qui contient en outre au moins une charge présente à l'état de particules.

12. Matériau dentaire conforme à l'une des revendications précédentes, qui contient
a) de 0,0001 à 5 % en poids, de préférence de 0,001 à 3,0 % en poids, et en particulier de 0,1 à 1,0 % en poids d'au moins un agent colorant photochrome encapsulé,
b) de 0,01 à 10 % en poids, et en particulier de 0,1 à 3,0 % en poids d'au moins un amorceur,
c) en option, de 5 à 85 % en poids, de préférence de 10 à 60 % en poids, et en particulier de 15 à 50 % en poids d'au moins un monomère,
d) en option, de 2,5 à 80 % en poids de charge(s),
e) et en option, de 2,5 à 70 % en poids de solvant,
par rapport, pour chacun, au poids total du matériau dentaire.

13. Matériau dentaire, conforme à la revendication 12, pour utilisation en tant qu'agent de colmatage de fissure, qui contient
a) de 0,0001 à 5 % en poids, de préférence de 0,001 à 3,0 % en poids, et en particulier de 0,1 à 1,0 % en poids d'au moins un agent colorant photochrome encapsulé,
b) de 0,01 à 10 % en poids, et en particulier de 0,1 à 3,0 % en poids d'au moins un amorceur,
c) en option, de 5 à 85 % en poids, de préférence de 10 à 60 % en poids, et en particulier de 15 à 50 % en poids d'au moins un monomère,
d) en option, de 2,5 à 80 % en poids de charge(s),
e) et en option, de 2,5 à 70 % en poids de solvant,
par rapport, pour chacun, au poids total du matériau dentaire.

14. Matériau dentaire, conforme à l'une des revendications 1 à 13, pour utilisation thérapeutique pour colmatage de fissures dentaires, pour restauration de dents endommagées, en tant que ciment dentaire, composite d'obturation dentaire ou matériau de placage.

15. Utilisation d'un agent colorant photochrome encapsulé, tel que défini dans l'une des revendications 1 à 8, pour la préparation d'un matériau dentaire photochrome.

16. Utilisation d'un matériau dentaire conforme à l'une des revendications 1 à 13 pour la fabrication ou la réparation extra-orale de restaurations dentaires telles que prothèses, dents artificielles, incrustations inlays ou onlays, couronnes, ou bridges.
